Europäisches Patentamt

European Patent Office

Office européen des brevets

(19)

(11) Veröffentlichungsnummer: **0 008 022**
B1

(12) **EUROPÄISCHE PATENTSCHRIFT**

(21) Anmeldenummer: **79102532.3**

(22) Anmeldetag: **18.07.79**

(51) Int. Cl.³: **C 07 C 45/00,**
**C 07 C 47/02,**
**C 07 C 47/228,**
**C 07 C 47/28**

(54) **Verfahren zur Herstellung von gesättigten aliphatischen, cycloaliphatischen und araliphatischen Aldehyden.**

(30) Priorität: **26.07.78 DE 2832699**

(43) Veröffentlichungstag der Anmeldung:
**20.02.80 Patentblatt 80/4**

(45) Bekanntmachung des Hinweises auf die Patenterteilung:
**25.03.81 Patentblatt 81/12**

(84) Benannte Vertragsstaaten:
**BE CH DE FR GB IT NL**

(56) Entgegenhaltungen:
**DE - A1 - 2 615 308**
**FR - A - 2 097 403**
**SU - A - 299 238**

**CHEMICAL ABSTRACTS, Band 69, Nr. 17, 21. Oktober 1968, (COLUMBUS, OHIO, USA) M. A. RYASHENTSEVA et al. "Catalytic properties of palladium-alumina catalysts containing rare-earth oxides" Seite 6213, Abstract Nr. 66 641 R**

(73) Patentinhaber: **BASF Aktiengesellschaft**
**Carl-Bosch-Strasse 38**
**D-6700 Ludwigshafen (DE)**

(72) Erfinder: **Heilen, Gerd, Dr. Chem.**
**Schifferstadter Strasse 1b**
**D-6720 Speyer (DE)**
(72) Erfinder: **Nissen, Axel, Dr.**
**Panoramastrasse 51**
**D-6906 Leimen (DE)**
(72) Erfinder: **Koernig, Wolfgang, Dr. Chem.**
**Dachsweg 5**
**D-6901 Dossenheim (DE)**
(72) Erfinder: **Horner, Michael, Dr. Chem.**
**Pfalzgrafenstrasse 15 b**
**D-6730 Neustadt (DE)**
(72) Erfinder: **Fliege, Werner, Dr. Chem. c/o BASF**
**Wyandotte Corp.**
**Central R+D P.O.Box 111**
**Wyandotte, Mich.48192 (US)**
(72) Erfinder: **Boettger, Guenther, Dr. Chem.**
**Langen Wingert 16 b**
**D-6702 Bad Duerkheim 1 (DE)**

Courier Press, Leamington Spa, England.

# 0 008 022

Verfahren zur Herstellung von gesättigten aliphatischen, cycloaliphatischen und araliphatischen Aldehyden

Die vorliegende Erfindung betrifft ein neues Verfahren zur Herstellung von gesättigten aliphatischen, cycloaliphatischen und araliphatischen Aldehyden durch selektive Hydrierung der entsprechenden olefinisch ungesättigten Aldehyde mit Wasserstoff an Palladium enthaltenden Katalysatoren in der Flüssigphase.

Die selektive, unter Erhaltung der Aldehydgruppe verlaufende Hydrierung olefinisch ungesättigter Aldehyde ist Gegenstand zahlreicher Untersuchungen und Verfahrensvorschriften. So ist es insbesondere aus der FR—PS 1 399 393 bekannt, Crotonaldehyd bei Normaldruck oder leicht erhöhtem Druck und bei Temperaturen zwischen 25 und 65°C an Palladium/Kohle und Palladium/Aluminiumoxid in der Flüssigphase quantitativ in Butyraldehyd zu überführen. Wenngleich diese milden Reaktionsbedingungen an sich wünschenswert sind, so bedingen sie doch für technische Synthesen den Nachteil relativ langer Reaktionszeiten. Bei schärferen Reaktionsbedingungen erweisen sich die genannten Katalysatoren indes zu aktiv und bewirken zum Teil auch eine Hydrierung der Aldehydgruppe.

Weiterhin ist es aus der DE—PS 1 941 634 bekannt, 2-Äthylhex-2-en-1-al bei 200 dar und 90°C mittels eines speziellen Palladium/Kieselsäure-Katalysators zum 2-Äthylhexan-1-al zu hydrieren. Dieser Katalysator ist jedoch verhältnismäßig wenig aktiv und läßt daher hinsichtlich der Reaktionszeiten trotz des erforderlichen hohen Druckes zu wünschen übrig.

Diesen und zahlreichen weiteren Verfahren zur partiellen Hydrierung olefinisch ungesättigter Aldehyde, bei denen verschiedenartige Katalysatoren unter verschiedenen Reaktionsbedingungen angewandt werden, ist ferner gemeinsam, daß sie nur in speziellen Fällen befriedigen und sich nicht allgemein anwenden lassen. Dies ist in technisch-wirtschaftlicher Hinsicht von erheblichem Nachteil, denn es erfordert die Herstellung und Lagerhaltung verschiedener Katalysatoren und meistens auch die Errichtung mehrerer unterschiedlicher Hydrieranlagen. Für chemische Großprodukte mit ganzjähriger Kapazitätsauslastung mag dieser Nachteil nicht ins Gewicht fallen, wohl aber, wie es für die Praxis häufig zutrifft, wenn es sich um mehrere Spezialprodukte handelt, die nur in geringeren Mengen benötigt werden. Hier wäre es wünschenswert, die verschiedenen Hydrierungen in nur einer Anlage unter weitgehend gleichen Reaktionsbedingungen und unter Verwendung nur eines Katalysators herzustellen.

Diesem Erfordernis entsprechend, lag der Erfindung die Aufgabe zugrunde, die Technik der in Rede stehenden Reaktion durch die Verwendung von universell anwendbaren, hoch wirksamen und gleichermaßen selektiven Katalysatoren zu bereichern. Speziell erstreckt sich diese Aufgabe auf die selektive Hydrierung der gegen die Perhydrierung besonders empfindlichen $\alpha,\beta$-ungesättigten Aldehyde zu den entsprechenden gesättigten Aldehyden.

Demgemäß wurde ein Verfahren zur Herstellung von gesättigten aliphatischen, cycloaliphatischen und araliphatischen Aldehyden durch selektive Hydrierung der entsprechenden olefinisch ungesättigten Aldehyde mit Wasserstoff an Palladium enthaltenden Katalysatoren in der Flüssigphase gefunden, welches dadurch gekennzeichnet ist, daß man hierzu ein Katalysatorsystem verwendet, dessen aktive Bestandteile aus 2—90 Gew.% Palladium und 10—98 Gew.% eines Oxids oder eines Salzes eines Seltenen Erdmetalls oder einer Mischung verschiedener derartiger Oxide und/oder Salze bestehen.

Katalysatorsysteme dieser Art sind in der DE—OS 26 15 308 beschrieben, wo sie für die Kondensation von Aldehyden mit Ketonen und die gleichzeitige Hydrierung der hierbei intermediär entstehenden ungesättigten Ketone empfohlen werden. Daß sich diese Katalysatorsysteme mit Erfolg auch zur selektiven Hydrierung olefinisch ungesättigter Aldehyde verwenden lassen, war nicht zu erwarten, da die Aldehydgruppe empfindlicher ist als die Ketogruppe und infolgedessen eine Perhydrierung der olefinisch ungesättigten Aldehyde zu den entsprechenden gesättigten Alkoholen zu befürchten war.

Als aktive Komponente neben dem Palladium eignen sich alle Oxide und Salze der Seltenen Erdmetalle (im folgenden als SE-Verbindungen bezeichnet), darunter vornehmlich die Oxide, und zwar besonders das Lanthanoxid ($La_2O_3$), das Samariumoxid ($Sm_2O_3$), das Gadoliniumoxid ($Gd_2O_3$) und das Holmiumoxid ($Ho_2O_3$) sowie vor allem das Ceroxid ($CeO_2$), das Praseodymoxid ($Pr_2O_3$) und das Neodymoxid ($Nd_2O_3$).

Anstelle der Oxide lassen sich erfindungsgemäß auch die Salze der Seltenen Erdmetalle verwenden, beispielsweise die Nitrate, Sulfate, Phosphate, Chloride und Carbonate. Bevorzugt werden indes die Salze organischer Säuren wie die Acetate, Propionate, Phenolate, Benzolsulfonate, Toluolsulfonate und besonders die organischen Salze der höheren Fettsäuren, z.B. der Stearinsäure. Sind die Salze in der zu hydrierenden Ausgangsverbindung löslich, so können sie dieser auch vor der Reaktion beigemischt werden, so daß diese Mischung nur noch über einen herkömmlichen Palladiumkontakt geleitet zu werden braucht. Diese Verfahrensweise empfiehlt sich besonders in den Fällen, in denen man die Hydrierung an einem bereits bestehenden herkömmlichen Palladiumkontakt ohne den Aufwand eines Katalysatorwechsels auf die erfindungsgemäße Hydrierung umstellen möchte. Das Verfahrensprodukt braucht dann von dem SE-Salz lediglich abdestilliert zu werden.

0 008 022

Im übrigen ist es nicht erforderlich, die reinen SE-Verbindungen zu verwenden, vielmehr eignen sich ebensogut deren Gemische, z.B. die handelsüblichen SE-Oxide und -Salze technischer Reinheit mit etwa 90 gew.%igem Anteil eines SE-Oxids bzw. -Salzes und dem Rest aus mehreren begleitenden anderen SE-Verbindungen.

Der Begriff Katalysatorsystem soll verdeutlichen, daß es lediglich auf die gleichzeitige Gegenwart der beiden Komponenten Palladium und SE-Verbindung während der Hydrierung ankommt. Demgemäß stellt sich die erfindungsgemäß erwünschte Wirkung bereits dann ein, wen Palladium und SE-Verbindung gemeinsam in einer Suspension des zu hydrierenden Aldehyds oder einer organischen Lösung dieses Aldehyds vorliegen. Ebenso verhält es sich, wenn es sich um eine Suspension eines Pd-Trägerkatalysators — z.B. mit Aktivkohle als Träger — und eines SE-Oxid-Trägerkatalysators — z.B. mit Aluminiumoxid als Träger — handelt.

Derartige Verfahrensweisen sind prinzipiell möglich und häufig auch für Hydrierungen im Labormaßstab oder im halbtechnischen Maßstab geeignet. Für den kontinuierlichen technischen Betrieb empfiehlt es sich jedoch aus allgemein bekannten verfahrenstechnischen Gründen, den Katalysator in einer Reaktorsäule fest anzuordnen und Wasserstoff und Aldehyd bzw. eine Lösung des Aldehydes über ein derartiges Festbett zu leiten.

Für diesen Zweck verwendet man vorzugsweise Trägerkatalysatoren, auf welche das Palladium und die SE-Verbindung gemeinsam aufgebracht sind. Man kann solche Trägerkatalysatoren herstellen, indem man das Trägermaterial mit einer wäßrigen Lösung, die ein Pd-Salz wie Palladiumnitrat sowie ein SE-Salz im entsprechenden Mengenverhältnis enthält, imprägniert, trocknet und im Luftstrom erhitzt, wobei sich das SE-Oxid bildet. Das Pd-Metall bildet sich dann unter den hydrierenden Bedingungen von selber, jedoch kann man auch den Trägerkatalysator zu diesem Zwecke einer gesonderten Hydrierung unterwerfen.

Als Trägermaterialen eignen sich z.B. Aktivkohle, Aluminiumoxid und Kieselgel in Form von Tabletten, Granalien, Kugeln und Strängen mit Durchmessern von 2—20 mm und einer Längenausdehnung von 5—50 mm.

Eine Schüttung von 1 Liter derartiger Trägerkatalysatoren enthält je nach Raumform und Gesamtoberfläche des Trägers etwa 4—80 g aktiver Katalysatorbestandteile.

Allgemein beträgt das Gewichtsverhältnis von Pd zur SE-Verbindung 2:98—90:10, jedoch sind in aller Regel solche Katalysatoren zu bevorzugen, in denen dieses Verhältnis zwischen 20:80 und 80:20 liegt.

Um 1 Mol des olefinisch ungesättigten Aldehydes bei 100°C und einem Wasserstoffdruck von 10 bar quantitativ oder praktisch quantitativ selektiv zu hydrieren, benötigt man je nach Katalysatorform etwa 2 bis 10 ml der vorstehend beschriebenen Trägerkatalysatoren, wobei die Reaktionszeit etwa 2—10 Stunden beträgt. Hierbei handelt es sich um Richtwerte, die sich nach bekannten Gesetzmäßigkeiten mit den Reaktionsbedingungen ändern, wie jeweils durch einige Versuche unschwer zu ermitteln ist. Von der Art des zu hydrierenden Aldehyds sind die Reaktionsbedingungen weithin unabhängig, so daß es das erfindungsgemäße Verfahren gestattet, in ein und derselben Anlage ohne Katalysatorwechsel verschiedenartige olefinisch ungesättigte Aldehyde zu hydrieren.

Das Verfahren gelingt mit gutem Erfolg bereits bei Normaldruck, jedoch kann zur Erhöhung der Reaktionsgeschwindigkeit auch ein Druck bis zu 100 bar angewendet werden. Noch höhere Drucke bringen im Verhältnis zum hierfür erforderlichen technischen Aufwand normalerweise keine Vorteile mehr. Im allgemeinen erzielt man die wirtschaftlich günstigsten Ergebnisse bei Drucken im Bereich von 1—50 bar.

Für die Reaktionstemperatur empfiehlt sich ein Bereich von 20—250, vorzugsweise 20—220°C. Bei tieferen Temperaturen als 20°C sinkt die Hydriergeschwindigkeit merklich ab, und höheren Temperaturen als 250°C werden durch Nebenreaktionen, insbesondere Aldolkondensationen, zunehmend Grenzen gesetzt. Bemerkenswerterweise findet indes auch mit höheren Temperaturen nur eine gerinfügige Zunahme der Hydrierung der Aldehyde zu den Alkoholen statt, sofern die Hydrierung der olefinischen Doppelbindung noch nicht beendet ist. Die Selektivität der erfindungsgemäßen Katalysatoren ist somit, was als besonderer Vorteil zu werten ist, weitgehend temperaturunabhängig.

Sofern die zu hydrierenden olefinisch ungesättigten Aldehyde unter den Reaktionsbedingungen flüssig sind, erübrigt sich die Mitverwendung eines Lösungsmittels, jedoch kann die Gegenwart eines Lösungsmittels auch in diesen Fällen insofern von Vorteil sein, als dadurch Nebenreaktionen wie Aldolkondensationen entgegengewirkt wird. Sind die Aldehyde fest, so sind sie in Lösung zu bringen.

Als Lösungsmittel eignen sich alle unter den Reaktionsbedingungen inerten Flüssigkeiten, beispielsweise $C_5$—$C_8$-Paraffine, Cyclohexan, Methanol, Äthanol, Isopropanol, Äthylacetat sowie Toluol oder Xylol.

Die Menge des Lösungsmittels ist nicht kritisch und beträgt üblicherweise das 0,5—10-fache der Menge des Aldehyds.

Das Verfahren kann auf alle olefinisch ungesättigten Aldehyde angewendet werden und unterliegt daher grundsätzlich keinen Beschränkungen. Tragen diese Aldehyde Substituenten, die ebenfalls hydriert werden könnten — z.B. Acylgruppen —, so ist deren Hydrierung nicht zu befürchten, zumal auch die Aldehydgruppe nicht angegriffen wird. Enthalten die Aldehyde mehrere olefinische Doppelbindungen, so werden diese Doppelbindungen alle abgesättigt.

3

0 008 022

Befindet sich die Aldehydgruppe in Konjugation zur Doppelbindung, so wird die Aldehydgruppe normalerweise besonders leicht angegriffen und zur Alkoholgruppe hydriert. Dieser unerwünschte Effekt, will man selektiv hydrieren, tritt beim erfindungsgemäßen Verfahren jedoch kaum auf, so daß es für diese Fälle, nämlich die Hydrierung $\alpha,\beta$-ungesättigter Aldehyde zu den entsprechenden gesättigten Aldehyden, die größte Bedeutung hat.

Technischer wichtig sind vor allem solche gesättigten Aldehyde, deren Kohlenwasserstoffrest 4 bis 20 C-Atome enthält. Als Ausgangsmaterialien für die Herstellung dieser Aldehyde seien beispielsweise folgende olefinisch ungesättigten Aldehyde genannt:

— aliphatische $\alpha,\beta$-olefinisch ungesättigte Aldehyde, wie Crotonaldehyd
2-Methylpent-2-en-1-al
2-Methylbut-2-en-1-al
3-Methylbut-2-en-1-al
2-Äthylhex-2-en-1-al
Citral (3,7-Dimethylocta-2,6-dien-1-al)
Citronellal (3,7-Dimethyloct-6-en-1-al)

— aliphatische olefinisch ungesättigte Aldehyde mit isolierter Doppelbindung, wie
3-Methylbut-3-en-1-ol,

— cycloaliphatisch olefinisch ungesättigte Aldehyde, wie
1-Formylcyclohex-3-en,

— araliphatisch olefinisch ungesättigte Aldehyd, wie Zimtaldehyd
$\alpha$-Methylzimtaldehyd
p-isopropyl-$\alpha$-methylzimtaldehyd
p-tert.-Butyl-$\alpha$-methylzimtaldehyd.

Die Verfahrensprodukte sind zum Teil wichtige Zwischenprodukte für Industriechemikalien, z.B. Butyraldehyd aus Crotonaldehyd für die Herstellung des Weichmacheralkohols 2-Äthylhexanol, 2-Äthylhexanal aus 2-Äthylhexenal für die Herstellung von 2-Äthylhexansäure sowie zum Teil für Duftstoffe aus der Aldehydreihe, beispielsweise 3,7-Dimethyloctanal aus Citral.

### Beispiel 1
### Herstellung verschiedener Trägerkatalysatoren

Jeweils 2400 g von 4 mm dicken Strängen eines Trägermaterials wurden mit 2500 ml einer salpetersauren wäßrigen Lösung getränkt, die soviel Palladiumnitrat und eines SE-Nitrats enthielt, wie der Zusammensetzung des fertigen Katalysators entsprach. Beim Tränken wurde die Lösung vollständig vom Trägermaterial aufgenommen. Nach dem Tränken wurden die Stränge 16 Stunden lang bei 120°C getrocknet und anschließend 6 Stunden lang auf 520°C erhitzt, wobei die SE-Nitrate in die SE-Oxide übergingen. Die Stränge wurden sodann in 2 cm lange Abschnitte gebrochen.

Die Charakteristiken der Katalysatoren gehen aus Tabelle 1 hervor.

4

# 0 008 022

TABELLE 1

Charakteristiken der verschiedenen Trägerkatalystoren gemäß Beispiel 1

| Katalysator | Träger | SE-Oxid | Gehalt in Gew.%, bezogen auf die Gesamtmenge des Katalysators an | | Verhältnis Pd:SE-Oxid |
|---|---|---|---|---|---|
| | | | Pd | SE-Oxid | |
| *erfindungsgemäß* | | | | | |
| A | $\gamma$-Al$_2$O$_3$ | Pr$_2$O$_3$ | 0,5 | 5,0 | 9:91 |
| B | $\gamma$-Al$_2$O$_3$ | Pr$_2$O$_3$ | 5,0 | 5,0 | 50:50 |
| C | $\gamma$-Al$_2$O$_3$ | CeO$_2$ | 0,1 | 5,0 | 2:98 |
| D | SiO$_2$ | Pr$_2$O$_3$ | 0,07 | 0,5 | 12:88 |
| *zum Vergleich* | | | | | |
| E | SiO$_2$ | — | 0,07 | — | — |
| F | $\gamma$-Al$_2$O$_3$ | — | 0,3 | — | — |
| G | $\gamma$-Al$_2$O$_3$ | — | 5,0 | — | — |

Beispiel 2

Hydrierung von 2-Äthylhex-2-en-1-al

In einem Rieselreaktor von 0,5 l Inhalt wurden in mehreren Versuchen unter den aus Tabelle 2 ersichtlichen Reaktionsbedingungen jeweils pro Stunde 50 g 2-Äthylhex-2-en-1-al hydriert. Die ebenfalls aus der Tabelle hervorgehenden Umsätze und Ausbeuten wurden gaschromatographisch bestimmt.

**0 008 022**

TABELLE 2

Reaktionsbedingungen und Ergebnisse der Versuche zu Beispiel 2

Hydrierung von 2-Äthylhex-2-en-1-al

| Versuch Nr. | Katalysator | Wasserstoffdruck (bar) | Temperatur (°C) | Umsatz (%) | Ausbeute hexanal, Umsatz (%) | 2-Äthyl- bezogen auf. absolut (%) | Ausbeute 2-Äthyl- hexanol absolut (%) |
|---|---|---|---|---|---|---|---|
| *erfindungsgemäß* | | | | | | | |
| 1 | A | 10 | 80 | 96 | 99 | 94 | in allen |
| 2 | A | 10 | 100 | 96 | 98 | 95 | Fällen <1 |
| 3 | A | 10 | 115 | 98 | 98 | 97 | |
| 4 | A | 15 | 80 | 99 | 99 | 98 | |
| 5 | A | 15 | 100 | 99 | 98 | 97 | |
| 6 | A | 15 | 115 | 100 | 98 | 98 | |
| 7 | A | 35 | 80 | 99 | 99 | 98 | |
| 8 | A | 35 | 100 | 98 | 98 | | |
| 9 | A | 35 | 115 | 100 | 98 | 98 | |
| 10 | C | 15 | 80 | 96 | 99 | 95 | |
| 11 | C | 15 | 100 | 97 | 99 | 96 | |
| 12 | C | 15 | 115 | 99 | 98 | 97 | |
| 13 | C | 15 | 150 | 100 | 97 | 97 | |
| 14 | D | 15 | 80 | 95 | 99 | 94 | |
| 15 | D | 15 | 100 | 97 | 99 | 96 | |
| 16 | D | 15 | 115 | 98 | 99 | 97 | |
| *herkömmlich* | | | | | | | |
| 17 | E | 15 | 100 | 40 | 92 | 37 | 2 |
| 18 | E | 35 | 100 | 52 | 92 | 48 | 3 |
| 19 | F | 35 | 115 | 50 | 98 | 45 | 3 |

**Beispiel 3**
**Hydrierung von Citral**
In einem Rührautoklaven von 300 ml Inhalt wurden jeweils 152 g Citral (3,7-Dimethylocta-2,6-dien-1-al) mit 3 g eines Katalysators solange hydriert, bis kein Wasserstoff mehr aufgenommen wurde, d.h., bis ein Umsatz von 100% erreicht war. Im einzelnen gehen die Versuchsbedingungen sowie die Verfahrensergebnisse aus Tabelle 3 hervor. Die Ausbeuten wurden gaschromatographisch bestimmt.

**Beispiele 4 bis 9**
**Hydrierung verschiedener ungesättigter Aldehyde**
Analog Beispiel 3 wurden jeweils 150 eines ungesättigten Aldehyds mit 3 g des Katalysators A solange hydriert, bis der Umsatz praktisch vollständig war. Die Verfahrensbedingungen sowie die Ergebnisse sind aus Tabelle 4 ersichtlich. Die Ausbeuten wurden gaschromatographisch ermittelt.

6

Beispiel 10

150 g einer 10 gew.%igen methanolischen Lösung von 4-tert.-Butyl-$\alpha$-methylzimtaldehyd wurden in Gegenwart eines Gemisches aus 1 g eines Pd-Aktivkohle-Katalysators mit 10 Gew.% Pd-Gehalt und 1 g Neodymstearat unter einem Wasserstoffdruck von 15 bar bei 110°C selektiv hydriert. Nach 6 Stunden war der Umsatz praktisch vollständig. Die gaschromatographisch bestimmte Ausbeute an 3-(4-tert.-Butylphenyl)-2-methylpropanal betrug 98%.

TABELLE 3

Reaktionsbedingungen und Ergebnisse der Versuche zu Beispiel 3

Hydrierung von Citral

| Versuch Nr. | Katalysator | Lösungsmittel | Wasserstoffdruck (bar) | Temperatur (°C) | Dauer (h) | Ausbeute (%) an | | |
|---|---|---|---|---|---|---|---|---|
| | | | | | | 3,7-Di-methyl-octanal | 3,7-Di-methyl-oct-6-en-1-al | 3,7-Di-methyl-octanol |
| erfindungsgemäß | | | | | | | | |
| 1 | B | ohne | 40 | 100 | 10 | 96 | 2 | <1 |
| 2 | B | ohne | 20 | 40 | 16 | 96 | 3 | <1 |
| 3 | B | 60 ml Methanol | 40 | 100 | 7 | 93 | 6 | <1 |
| herkömmlich | | | | | | | | |
| 4 | G | 60 ml Methanol | 40 | 100 | 7 | 20 | 66 | 4 |

## TABELLE 4

Hydrierung verschiedener ungesättigter Aldehyde

Beispiel 4—9

| Beispiel Nr. | ungesätt. Aldehyd | Wasserstoffdruck (bar) | Temperatur (°C) | Dauer (h) | Ausbeute in (%) an | |
|---|---|---|---|---|---|---|
| | | | | | gesättigtem Aldehyd | gesättigtem Alkohol |
| 4 | 2-Methyl-pent-2-en-1-al | 20 | 160 | 3 | 95 | 1 |
| 5 | 3,7-Dimethyloct-6-en-1-al | 40 | 100 | 8 | 98 | 1 |
| 6 | Formylcyclohex-3-en | 20 | 130 | 6 | 95 | 2 |
| 7 | $\alpha$-Methylzimtaldehyd | 17 | 120 | 7 | 97 | 1 |
| 8 | 4-tert.-Butyl-$\alpha$-methyl-zimtaldehyd | 20 | 100 | 8 | 97 | 1 |
| 9 | 4-Isopropyl-$\alpha$-methyl-zimtaldehyd | 20 | 110 | 7 | 98 | 1 |

**0 008 022**

**Patentanspruch**

Verfahren zur Herstellung von gesättigten aliphatischen, cycloaliphatischen und araliphatischen Aldehyden durch selektive Hydrierung der entsprechenden olefinisch ungesättigten Aldehyde mit Wasserstoff an Palladium enthaltenden Katalysatoren in der Flüssigphase, dadurch gekennzeichnet, daß man hierzu ein Katalysatorsystem verwendet, dessen aktive Bestandteile aus 2—90 Gew.% Palladium und 10—98 Gew.% eines Oxids oder eines Salzes eines Seltenen Erdmetalls oder einer Mischung verschiedener derartiger Oxide und/oder Salze bestehen.

**Revendication**

Procédé de préparation d'aldéhydes aliphatiques, cycloaliphatiques et araliphatiques saturés par hydrogénation sélective des aldéhydes à insaturation oléfinique correspondants en phase liquide par l'hydrogène en présence d'un catalyseur au palladium, caractérisé en ce que l'on emploie un système catalytique, dont les composants actifs sont constitués de palladium en une proportion de 2 à 90% en poids et d'un oxyde ou d'un sel d'un métal terreux rare ou d'un mélange de plusieurs oxydes et (ou) sels de métaux terreux rares en une proportion de 10 à 98% en poids.

**Claim**

A process for the preparation of saturated aliphatic, cycloaliphatic and araliphatic aldehydes by selective hydrogenation of the corresponding olefinically unsaturated aldehydes with hydrogen in the presence of a palladium-containing catalyst in the liquid phase, wherein use is made of a catalyst system whose active constituents consist of from 2 to 90% by weight of palladium and from 10 to 98% by weight of an oxide or salt of a rare earth metal or a mixture of such oxides and/or salts.